## Europäisches Patentamt

(19) **European Patent Office**

**Office européen des brevets**

(11) Veröffentlichungsnummer: **0 173 828**

**B1**

(12) # EUROPÄISCHE PATENTSCHRIFT

(45) Veröffentlichungstag der Patentschrift:
27.04.88

(21) Anmeldenummer: **85108774.2**

(22) Anmeldetag: **13.07.85**

(51) Int. Cl.⁴: **C 07 C 19/02,** C 07 C 17/04

(54) Verfahren zur Herstellung von 1,2,3-Trichlorpropan.

(30) Priorität: **06.09.84 DE 3432720**

(43) Veröffentlichungstag der Anmeldung:
**12.03.86 Patentblatt 86/11**

(45) Bekanntmachung des Hinweises auf die Patenterteilung:
**27.04.88 Patentblatt 88/17**

(84) Benannte Vertragsstaaten:
**AT BE CH DE FR GB IT LI NL**

(56) Entgegenhaltungen:
**EP-A-0 133 913**
**DE-B-1 240 057**
**DE-C-894 554**
**US-A-2 302 228**

(73) Patentinhaber: **HÜLS AKTIENGESELLSCHAFT, -
RSP Patente / PB 15 - Postfach 13 20, D-4370
Marl 1 (DE)**

(72) Erfinder: **Müller, Dieter Jürgen, Dr., Stargarder
Strasse 30, D-4370 Marl (DE)**
Erfinder: **Wehmeyer, Heinrich, An der Tränke 20,
D-4370 Marl (DE)**

EP 0 173 828 B1

**Beschreibung**

Die Erfindung betrifft ein Verfahren zur Herstellung von 1,2,3-Trichlorpropan durch Umsetzung von Allylchlorid mit Sulfurylchlorid in Gegenwart eines Katalysators.

Die Chlorierung von Allylchlorid zu 1,2,3-Trichlorpropan mit Sulfurylchlorid gelingt nach dem Stand der Technik nur in Gegenwart von katalytisch wirkenden Mengen an peroxidischen Verbindungen (Am, Soc, 61 (1939) 2145; US-PS 2 302 228; Am. Soc, 61 (1939) 3433). Diese radikalische Chlorierung verläuft allerdings trotz vierfachen Überschusses an Allylchlorid wenig selektiv, so daß beispielsweise bei Gesamtausbeuten an Chlorierungsprodukten von 80 % nur 37 % als 1,2,3-Trichlorpropan resultieren (US-PS 2 302 228, Beispiel 4). Höhere Ausbeuten sind erst nach erheblicher Verdünnung des Reaktionsgemisches mit beispielsweise CCl$_4$ im Gewichtsverhältnis von wenigstens 1 : 1 erreichbar, wobei ebenfalls nur in Gegenwart von peroxidischen Verbindungen Ausbeuten von 80 bis 90 % an 1,2,3-Trichlorpropan erhalten werden (Am, Soc, 61 (1939) 3433).

Nach der DE-B-1 240 057 erhält man durch Chlorieren von Butadien mit Chlor 1,2,3,4-Tetrachlorbutan ohne wesentliche Bildung von Trichlorbutan. Die Umsetzung erfolgt in einem inerten Lösemittel, das zwischen 0,1 und 20 Gewichtsprozent Pyridin als Katalysator enthält. Der Katalysatoreinsatz ist somit sehr hoch. Nach den Beispielen entstehen zudem 10 Gewichtsprozent höherchlorierte Substitionsprodukte.

Diese Verfahren sind wegen zu geringer Selektivität bzw. zu geringer Raumzeitausbeute technisch unbefriedigend.

Hieraus ergab sich die Aufgabe, ein Verfahren bereizustellen, das es gestattet, die Umsetzung von Allylchlorid mit Sulfurylchlorid ohne notwenige Verdünnung und ohne extremen überstöchiometrischen Einsatz von Allylchlorid mit hoher Selektivität und hoher Raumzeitausbeute herzustellen.

Diese Aufgabe ist erfindungsgemäß entsprechend den Angaben der Patentansprüche gelöst worden.

Überraschenderweise ist gefunden worden, daß die Umsetzung von Allylchlorid mit Sulfurylchlorid auch ohne Verdünnung mit inerten Lösemitteln und ohne extreme Überschüsse an Allylchlorid mit unerwartet hoher Ausbeute von 80 bis 90 % zu 1,2,3-Trichlorpropan führt, wenn die Umsetzung in Gegenwart von katalytisch wirkenden Mengen an Aminen und/oder phosphinen bzw. Phosphinoxiden durchgeführt wird.

Diese überraschende Reaktion verläuft offensichtlich nicht über den vorbeschriebenen radikalischen, sondern vermutlich über einen bisher nicht bekannt gewordenen ionischen Mechanismus nach folgender Bruttogleichung:

$$CH_2 = CH-CH_2Cl + SO_2Cl_2 \xrightarrow[\text{bzw. Phosphinoxid}]{\text{Amin/Phosphin}} CH_2-CH-CH_2 + SO_2$$
$$\qquad\qquad\qquad\qquad\qquad\qquad\qquad\qquad\quad\; Cl \quad Cl \; Cl$$

Die Umsetzung wird erfindungsgemäß in der Weise durchgeführt, daß man Allylchlorid, vorzugsweise in der Flüssigphase, in Gegenwart von Aminen und/oder Phosphinen bzw. Phosphinoxiden im Temperaturbereich von etwa 30°C bis etwa 70°C durch Zugabe von SO$_2$Cl chloriert. Die Chlorierungsreaktion verläuft exotherm mit großer Geschwindigkeit, so daß es für eine ruhige Reaktionsführung zweckmäßig ist, die Umsetzung in der Weise durchzuführen, daß man dem Allylchlorid nach Zugabe der Amine bzw. der Phosphine und Aufheizen auf eine Starttemperatur von etwa 30 bis 40°C Sulfurylchlorid unter Kühlung in dem Maße zudosiert, wie es abreagiert. Die Reaktionstemperatur kann dabei bis an die Siedetemperatur der Reaktionsmischung heranreichen, wobei auch unter Druck gearbeitet werden kann, um die Reaktionstemperatur anzuheben. Die Abfuhr der Reaktionswärme kann sowohl über eine Mantelkühlung als auch über eine Verdampfungs-/Rückfluß-Kühlung erfolgen. Entsprechend der Reaktionsgleichung entsteht aus Sulfurylchlorid im wesentlichen das gasförmige Nebenprodukt SO$_2$, das laufend aus der Reaktionsmischung ausgast und abgeführt wird.

Zur Umsetzung kann Sulfurylchlorid und Allylchlorid im stöchiometrischen Mengenverhältnis eingesetzt werden. Zweckmäßigerweise wählt man jedoch einen stöchiometrischen Unterschuß an SO$_2$Cl$_2$, da nicht umgesetztes Allylchlorid leichter aus dem resultierenden Produktgemisch abtrennbar ist als nicht umgesetztes Sulfurylchlorid. Nicht umgesetztes Allylchlorid kann nach Abtrennung erneut eingesetzt werden. Allerdings ist es für die Reaktion selbst auch nicht nachteilig, wenn SO$_2$Cl im stöchiometrischen Überschuß eingesetzt wird.

Die Reaktion kann sowohl diskontinuierlich, beispielsweise in einem Rührkessel, als auch kontinuierlich, beispielsweise in einem Rohrreaktor oder einer Kaskade durchgeführt werden. Bevorzugt führt man die Umsetzung in der Flüssigphase durch.

Als Katalysatoren für die Chlorierungsreaktion sind sowohl aliphatische, aromatische und heterocyclische Amine bzw. Stickstoffbasen wie Diisopropylamin, Triethylamin, Diphenylamin, Pyridin, Picoline, Pyrrol, Pyrazol und Chinolin als auch aliphatische oder aromatische Phosphine wie Tributylphosphin und Triphenylphosphin geeignet. Ebenso sind Kombinationen von Aminen und/oder Phosphinen einsetzbar. Außerdem sind auch Phosphinoxide, wie beispielsweise Triphenylphosphinoxid als Katalysatoren geeignet.

Bevorzugt setzt man Pyridin und/oder Tributylphosphin ein.

In der Regel genügen 1 bis 10 000 ppm dieser Verbindungen, um die Reaktion in der gewünschten Weise zu katalysieren. Bevorzugt setzt man 10 bis 1 000 ppm ein.

2

Da Chlorkohlenwasserstoffe teilweise mit Aminen stabilisiert werden, kann ggf. auf diese Weise stabilisiertes Allylchlorid bereits in der gewünschten Weise reagieren, ohne daß die zusätzliche Zugabe eines Amins oder Phosphins erforderlich wird.

Wenn auf die Zugabe eines Amins oder Phosphins zu Allylchlorid verzichtet wird, gelingt die Umsetzung zwar auch noch bei N-Gehalten von etwa 1 ppm, doch verläuft die Reaktion dann langsamer und kann außerdem eine unerwünschte Induktionsperiode aufweisen. Dadurch wird ein gleichmäßiger, kontrollierter Reaktionsablauf erschwert, so daß bevorzugt mit Zugabe von wenigstens 10 ppm Amin oder Phosphin bzw. Phosphinoxid gearbeitet wird.

Die Umsetzung erfolgt im allgemeinen unter Lichtausschluß bei 30° bis 70°C unter Normaldruck bis etwa 1,5 bar, vorzugsweise bei der Siedetemperatur der Reaktionsmischung, die bei Normaldruck von anfänglich etwa 42°C bis auf etwa 50°C steigt.

Die Zudosierzeit von $SO_2Cl_2$ beträgt im allgemeinen 3 bis 15 min; die Reaktionszeit bis zur Beendigung der $SO_2$-Entwicklung beträgt im allgemeinen 60 bis 120 min.

Zur Isolierung des 1,2,3-Trichlorpropans wird das Rohprodukt - ggf. nach einer Wasserwäsche und Trocknung - einer üblichen Fraktionierung, vorzugsweise unter vermindertem Druck, unterworfen, da sich das Produkt unter thermischer Belastung teilweise zersetzen kann.

Die Ausbeuten des erfindungsgemäßen Verfahrens an 1,2,3-Trichlorpropan liegen im allgemeinen bei 80 bis 90 %, bezogen auf umgesetztes Allylchlorid, wobei der Umsatz von im Unterschuß eingesetztem $SO_2Cl_2$ vollständig ist. Die Ausbeuten an 1,2,3-Trichlorpropan variieren etwas mit der Temperaturführung, der Zudosiergeschwindigkeit von Sulfurylchlorid, dem stöchiometrischen Verhältnis der Reaktionskomponenten, der Wahl des Katalysators und der Reaktionszeit. Als Nebenprodukt entsteht im wesentlichen ein sulfoniertes Produkt, dessen Bildung durch Lichteinwirkung begünstigt wird. Das unerwünschte Nebenprodukt kann leicht auf extraktivem Wege durch Wasserwäsche abgetrennt werden.

Die folgenden Beispiele sollen das erfindungsgemäße Verfahren verdeutlichen, ohne daß die gewählten Parameter im einzelnen als bindend anzusehen sind.

**Beispiele**

In einem temperierbaren Doppelmantelgefäß mit Magnetrührer und Rückflußkühler werden 1 mol Allylchlorid vorgelegt, auf die Starttemperatur von 30°C bzw. die Reaktionstemperatur erwärmt und nach Zugabe des Katalysators mit 0,8 mol Sulfurylchlorid unter Lichtausschluß umgesetzt. Die Temperatur im Reaktor wird durch Mantel- und Rückflußkühlung weitgehend konstant gehalten.

Das Sulfurylchlorid wird mittels einer Pumpe gleichmäßig innerhalb von etwa 15 min zudosiert, wobei der $SO_2Cl_2$-Eintritt unterhalb des Flüssigkeitsspiegels erfolgt.

Das bei der Umsetzung entstehende gasförmige $SO_2$, ggf. mit etwas HCl, entweicht über den Kühler und - falls erforderlich - durch ein Druckhalteventil in eine Vorlage mit verdünnter Natronlauge.

Nach Beendigung der Reaktion, d. h. wenn die Gasentwicklung zum Stillstand kommt, wird das Reaktionsgemisch nach Abkühlen auf 25°C einer Wasserwäsche und Trocknung mit $CaCl_2$ unterworfen. Das getrocknete Produktgemisch wird gaschromatografisch analysiert.

Die Ergebnisse dieser Versuchsdurchführung sind in Tabelle 1 aufgeführt.

**Tabelle 1**

| Katalysatorzugabe | | Reaktions-temperatur | Reaktions-zeit | Produktmenge nach Wasserwäsche und Trocknung (g) | Gehalt an 1,2,3-Trichchlorpropan (ohne überschüssiges Allylchlorid) | Ausbeute |
|---|---|---|---|---|---|---|
| | ppm | °C | min | | % | % |
| Pyridin | 1 000 | 45 | 90 | 108 | 95,5 | 87 |
| Pyridin | 10 | 40 | 120 | 108 | 92,0 | 84 |
| Tributylamin | 100 | 45 | 90 | 107 | 93,5 | 85 |
| Diphenylamin | 100 | 50 | 90 | 104 | 91,5 | 81 |
| Tributyl-phosphin | 100 | 50 | 60 | 110 | 96,5 | 90 |
| Triphenyl-phosphinoxid | 1 000 | 45 | 70 | 107 | 90,5 | 82 |
| *) | | 50 bis 60 | 150 | 105 | 90,0 | 80 |

*) Das eingesetzte Allylchlorid hatte einen N-Gehalt von ca. 1 ppm.

**0 173 828**

## Patentansprüche

1. Verfahren zur Herstellung von 1,2,3-Trichlorpropan durch Umsetzung von Allylchlorid mit Sulfurylchlorid, dadurch gekennzeichnet, daß man die Umsetzung in Gegenwart von 1 bis 10 000 ppm an Aminen bzw. Stickstoffbasen, Phosphinen und/oder Phosphinoxiden durchführt.

2. Verfahren nach Anspruch 1, dadurch gekennzeichnet, daß man die Reaktion in der Flüssigphase durchführt.

3. Verfahren nach den Ansprüchen 1 und 2, dadurch gekennzeichnet, daß man die Reaktion bei 30 bis 70°C durchführt.

4. Verfahren nach den Ansprüchen 1 bis 3, dadurch gekennzeichnet, daß man die Reaktion bei der Siedetemperatur des Reaktionsgemisches durchführt.

5. Verfahren nach den Ansprüchen 1 bis 4, dadurch gekennzeichnet, daß man Sulfurylchlorid, bezogen auf Allylchlorid, im stöchiometrischen Unterschuß einsetzt.

6. Verfahren nach den Ansprüchen 1 bis 5, dadurch gekennzeichnet, daß man die Umsetzung unter Lichtausschluß durchführt.

7. Verfahren nach den Ansprüchen 1 bis 6, dadurch gekennzeichnet, daß man als Amine aliphatische, heterocyclische oder aromatische Amine bzw. Stickstoffbasen einsetzt.

8. Verfahren nach den Ansprüchen 1 bis 6, dadurch gekennzeichnet, daß man als Phosphine aliphatische oder aromatische Phosphine oder deren Derivate in Form ihrer Oxide einsetzt.

## Claims

1. A process for the production of 1,2,3-trichloropropane through reaction of allyl chloride with sulphuryl chloride, characterised in that the reaction is carried out in the presence of 1 to 10,000 ppm of amine or nitrogen base, phosphine and/or phosphine oxide.

2. A process according to claim 1, characterised in that the reaction is carried out in the liquid phase.

3. A process according to any of claims 1 to 2, characterised in that the reaction is carried out at 30 to 70°C.

4. A process according to any of claims 1 to 3, characterised in that the reaction is carried out at the boiling point of the reaction mixture.

5. A process according to any of claims 1 to 4, characterised in that sulphuryl chloride is employed in a stoichiometric deficiency, relative to allyl chloride.

6. A process according to any of claims 1 to 5, characterised in that the reaction is carried out under exclusion of light.

7. A process according to any of claims 1 to 6 characterised in that there are employed as amines aliphatic, heterocyclic or aromatic amines or nitrogen bases.

8. A process according to any of claims 1 to 6, characterised in that there are employed as phosphines aliphatic or aromatic phosphines or derivatives thereof in the form of their oxides.

## Revendications

1. Procédé de préparation de 1,2,3-trichloro-proane par réaction de chlorure d'allyle sur du chlorure de sulfuryle, caractérisé par le fait que l'on effectue la réaction en présence de 1 à 10 000 ppm d'amines ou de bases azotées, de phosphines et/ou d'oxydes de phosphine.

2. Procédé selon la revendication 1, caractérisé par le fait que l'on effectue la réaction en phase liquide.

3. Procédé selon les revendications 1 et 2, caractérisé par le fait que l'on effectue la réaction à une température de 30 à 70°C.

4. Procédé selon les revendications 1 à 3, caractérisé par le fait que l'on effectue la réaction à la température d'ébullition du mélange réactionnel.

5. Procédé selon les revendications 1 à 4, caractérisé par le fait que relativement au chlorure d'allyle, on utilise le chlorure de sulfuryle dans une quantité inférieure à la quantité stoechiométrique.

4

6. Procédé selon les revendications 1 à 5, caractérisé par le fait que l'on effectue la réaction à l'abri de la lumière.

7. Procédé selon les revendications 1 à 6, caractérisé par le fait que l'on utilise, comme amines, des amines aliphatiques, hétérocycliques ou aromatiques, ou des bases azotées.

8. Procédé selon les revendications 1 à 6, caractérisé par le fait que l'on utilise, comme phosphines, des phosphines aliphatiques ou aromatiques, ou leurs dérivés sous la forme de leurs oxydes.